# EUROPEAN PATENT SPECIFICATION

(11) **EP 1 793 894 B1**
(45) Date of publication and mention of the grant of the patent: **02.06.2010**
(21) Application number: 05782901.2
(22) Date of filing: 12.09.2005
(51) Int. Cl.: A61N 1/39

(54) **EXTERNAL DEFIBRILLATOR HAVING A CERAMIC STORAGE CAPACITOR AND ENERGY CONDITIONING CIRCUIT**
EXTERNER DEFIBRILLATOR MIT KERAMIKSPEICHERKONDENSATOR UND ENERGIEKONDITIONIERUNGSSCHALTUNG
DEFIBRILLATEUR EXTERNE AYANT UN CONDENSATEUR DE MEMOIRE CERAMIQUE ET UN CIRCUIT DE CONDITIONNEMENT D'ENERGIE

(30) Priority: 20.09.2004 US 611331 P
(43) Date of publication of application: 13.06.2007
(73) Proprietor: Koninklijke Philips Electronics N.V., 5621 BA Eindhoven (NL)
(72) Inventor: HUGH, Steven, C., Bothell, WA 98041-3003 (US); POWERS, Daniel, J., Bothell, WA 98041-3003 (US)
(74) Representative: Schouten, Marcus Maria
(86) International application number: PCT/IB2005/052982
(87) International publication number: WO 2006/033043

(56) References cited:
- US-A- 5 337 209
- US-A- 5 545 184
- US-A- 5 808 856
- US-A1- 2003 216 786

## Description

This invention generally relates cardiac defibrillators, and more particularly, to external defibrillators employing high -voltage ceramic capacitors to store the electrical energy that is delivered to a patient.

Sudden cardiac arrest (SCA) most often occurs without warning, striking people with no history of heart problems. It is estimated that more than 1000 people per day are victims of sudden cardiac arrest in the United States alone. SCA results when the electrical component of the heart no longer functions properly causing an abnormal sinus rhythm. One such abnormal sinus rhythm, ventricular fibrillation (VF), is caused by abnormal electrical activity in the hea rt. As a result, the heart fails to adequately pump blood through the body. VF may be treated by applying an electric shock to a patient's heart through the use of a defibrillator.

Defibrillators include manual defibrillators, automatic or semi -automatic external defibrillators (AEDs), defibrillator/monitor combinations, advisory defibrillators and defibrillator trainers. A defibrillator shock clears the heart of abnormal electrical activity (in a process called "defibrillation") by producing a momentary asystole and providing an opportunity for the heart's natural pacemaker areas to restore normal rhythmic function. Currently available external defibrillators provide either a monophasic or biphasic electrical pulse to a patient through electrodes applied to the chest. Monophasic defibrillators deliver an electrical pulse of current in one direction, whereas biphasic defibrillators deliver an electrical pulse of current first in one direction and then in the opposite direction. When delivered external to the patient, these electrical pulses are high -voltage, high-energy pulses, typically in excess of 1000 volts and in the range of 100 to 300 Joules of energy.

Of the wide variety of external defibrillators currently available, AEDs are becoming increasingly popular because they can be used by relatively inexperienced personnel. Additionally, these external defibrillators can be made relatively lightweight, compact, and portable, such as those used by paramedics and EMS personnel, or attached to carts such as those found in clinics and hospitals. However, the portability of a defibrillator is limited by hardware and design constraints. For example, with respect to design constraints, conventional design rules for high -energy and high-voltage systems, such as are used in an external defibrillalor, dictate that the high-voltage components of the defibrillator be spaced apart by a minimum distance requirement. As a result, the physical size of the defibrillator is affected since the defibrillator case must be suffrcient to accommodate the minimum spacing design rule.

With respect to hardware constraints, various components of the defibrillator are seiected for their stability over a wide range of environmental operating conditions, such as varying temperature and humidity. One such component is a storage capacitor of the defibrillator, which typically is used to store electrical energy that is eventually delivered to a patient as a defibrillating pulse. As previously mentioned, the defibrillating pulses can be in excess of 1000 volts and are typically in the range of hundreds of Joules of energy. Consequently, the storage capacitor of a defibrillator typically has a capacitance between 100 and 200 pF and is rated for approximately 2000 volts. The storage capacitors are further selected based on the ability to maintain stable capacitance characteristics over a wide range of temperatures since the AEDs are deployed over such a wide range of different environments such as those encountered by emergency rescue vehicles in a variety of different climates. Conventional storage capacitors are typically film or electrolytic capacitors that are several cubic inches in volume. The resulting storage capacitors, which have sufficient capacitance and voltage characteristics, as well as suitable stability over various environmental operating conditions, have physical dimensions which constitute a significant portion of an AEDs overall size. As a result, minimizing the overall size of the defibrillator will be limited by the physical dimensions of a typical storage capacitor. Therefore, to facilitate reducing the physical size ofan external defibrillator, an alternative design is desirable.

US 5545184 discloses a cardiac defibrillator which includes a multilayer capacitor with a plurality of conductive electrodes and interspersed dielectric layers, each dielectric layer composed of constituents which cause the dielectric layer to exhibit antiferroelectric characteristics.

US 5808856 discloses a high energy multilayer ceramic capacitor formed of alternating ceramic and electrode layers, the capacitor being suitable for use in implantable medical devices.

US 2003/216786 discloses a circuit for producing an arbitrary defibrillator waveform using switching techniques which reduce the usual high current or high voltage stress on the switching element. This allows the use of a smaller energy storage capacitor for a given delivered energy.

The problem with these references is that temperature dependency and nonlinear capacitance characteristics of the ceramic storage capacitors used cannot be controlled.

This problem is solved by an external defibrilator according to claim 1.

The present invention is directed to an external defibriltator for provid ing defibrillating pulses to a patient including a ceramic storage capacitor and an energy conditioning circuit. The ceramic storage capacitor has an electrical discharge characteristic and is electrically charged by a charging circuit coupled to the cera mic storage capacitor. The energy conditioning circuit is also coupled to the ceramic storage capacitor and is configured to receive the electrical energy discharging according to the electrical discharge characteristic from the ceramic storage capacitor and, in response, provide the electrical energy according to a modified electrical discharge characteristic. A steering circuit coupled to the energy conditioning circuit is configured to couple the electrical energy discharging according to the modified electrical discharge characteristic to a pair of electrodes to deliver a defibrillating pulse having a defibrillating pulse characteristic to the patient.

### BRIEF description of the drawings

Figure 1 is a functional block diagram of an external defibrillat or according to an embodiment of the present invention.
Figure 2 is a functional block diagram of a high -voltage delivery circuit of the external defibrillator of Figure 1.
Figure 3 is a functional block diagram of an energy storage circuit of the high - voltage delivery circuit of Figure 2.
Figure 4 is a top plan view and a side view of a ceramic storage capacitor of the energy storage circuit of Figure 3.
Figures 5A-5C are schematic drawings of various energy conditioning circui ts that can be utilized in the energy storage circuit of Figure 3.

Embodiments of the present invention are directed to an external defibrillator that includes a ceramic storage capacitor and an energy conditioning circuit for conditioning the discharge of the ceramic capacitor. The physical size of the resulting external defibrillator can be reduced over conventional external defibrillators since ceramic capacitors are typically more compact for a given capacitance than their film and electrolytic counterparts. Ceramic capacitors, however, are subject to wide variation in capacitance value with temperature changes. This characteristic has limited the use of ceramic capacitors to indwelling defibrillators where the ceramic capacitor temperature can be expected to remain within a few degrees of normal body temperature of 98.6°F. This characteristic has heretofore caused ceramic capacitors to be seen as unacceptable for use in external defibrillators. However, an energy conditioning circuit is included in embodiments of the present invention to accommodate performance shortcomings of ceramic capacitors that have prevented utilization of ceramic capacitors in external defibrillators. In the following description certain specific details are set forth in or der to provide a thorough understanding of embodiments of the present invention. It will be clear, however, to one skilled in the art, that the present invention can be practiced without these details. In other instances, well-known circuits have not bee n shown in detail in order to avoid unnecessarily obscuring the description of the various embodiments of the invention. Also not presented in any great detail are those well -known control signals and signal timing protocols associated with the internal operation of defibrillators.

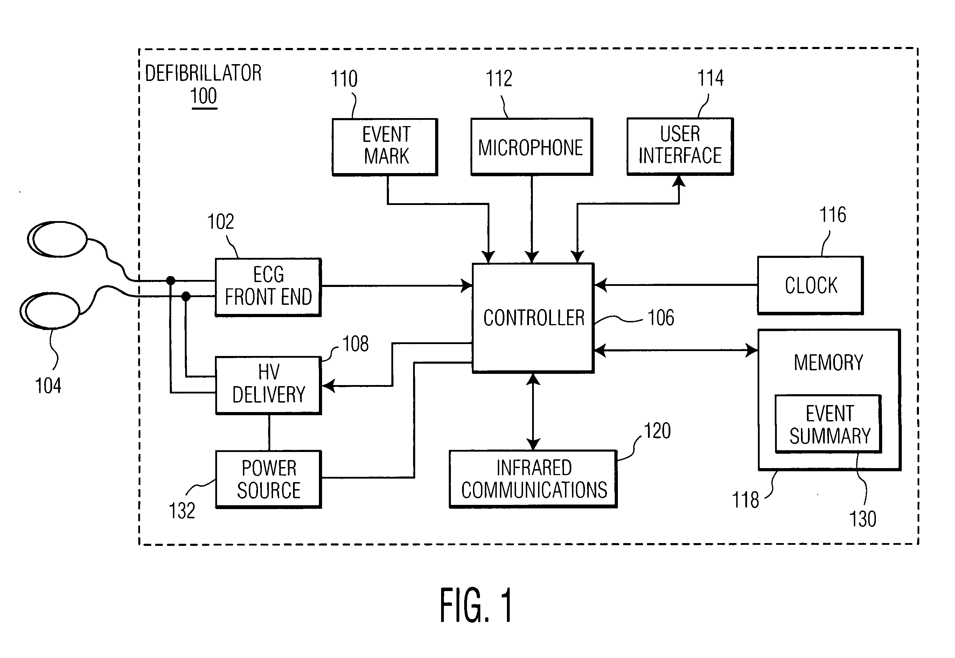

Figure 1 is a schematic representation of the defibrillator 100. A pair of electrodes 104 are included to provide a defibrillating pulse when attached to a patient. The electrodes 104 further provide an ECG signal to the ECG front end 102, which provides the ECG signal to a controller 106 for evaluation and display to the operator via a user interface 114. The ECG information can also be stored by the controller 106 in a memory 118. The memory 118 can also be used to store an event summary 130, in which information from an event mark 110, a microphone 112, and/or from a clock 116 are stored. The event summary information is useful during a transfer (often called a handoff) between an emergency medical technician and a hospi tal in order to continue the treatment of the patient. An infrared communications port 120 is also included in the defibrillator 100 to communicate the information in the memory 118 with an outside device during the transfer.

A power source 132 included in the defibrillator 100 provides power to the entire defibrillator 100. The power source 132 can be a line source or a battery, or any similar device which provides sufficient power for a defibrillating pulse and the ECG monitoring functions described herein. The power source 132 is typically a disposable or rechargeable battery for portable external defibrillators such as the defibrillator 100. A high voltage (HV) delivery circuit 108 administers a defibrillating pulse to the patient via the electrodes 104 at the command of the controller 106. At the instigation of the operator using a shock button (not shown), the charge from the high voltage delivery device 108 is administered to the patient in order to bring about the normal rhythmic ventricular contractions. The power source 132 supplies the charging energy to the high voltage delivery device 108 during a charging time in order to store sufficient energy to administer a treatment. The charging time is preferably small since the rapid administration of the treatment is desirable to produce a favorable result.

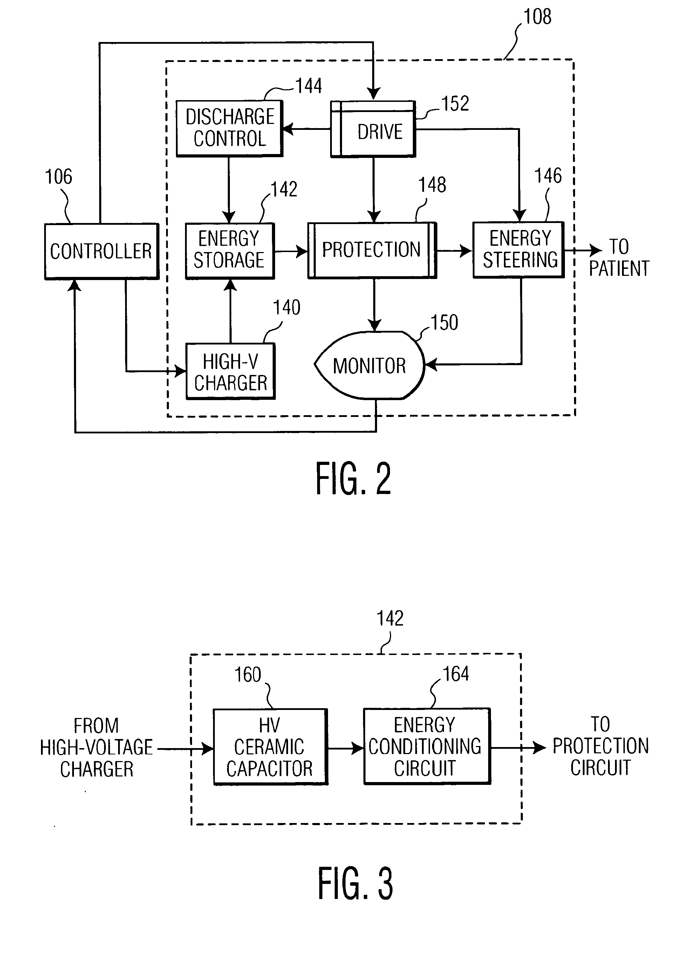

As shown in Figure 2, the high -voltage delivery circuit 108 includes a number of functional circuit blocks which are both monitored and controlled by the controller 106. A high-voltage charging circuit 140, such as a flyback power supply, responds to one or more control signals issued by the controller 106 and generates electrical energy for provision to an energy storage circuit 142. The storage circuit 142 stores the electrical energy for subsequent delivery to the patient. A discharge control circuit 144 controls discharge of the energy stored in the energy storage circuit 142 to an energy transfer or steering circuit 146 through a protection circuit 148. The steering circuit 146 in tur n delivers the electrical energy to the patient via the electrodes 104 (Figure 1). The steering circuit 146 may deliver the electrical energy to the patient with a single polarity (e.g., a monophasic pulse) or with an alternating polarity (e.g., a biphasic or multiphasic pulse), as required by the desired implementation. In one embodiment of the present invention, the energy steering circuit 146 is has an "H -bridge" configuration, with four switching elements (not shown), such as silicon controlled rectifiers (SCRs). The switching of the four switching elements to deliver a defibrillating pulse, monophasic or biphasic, is under the control of a drive circuit 152.

The protection circuit 148 functions to limit energy delivery from the energy storage circu it 142 to the steering circuit 146 and to discharge or otherwise disarm the energy storage circuit 142 in the event of a fault condition. The protection circuit 148 operates to limit the time -rate-of-change of the current flowing through the steering circuit 146. A monitor circuit 150 senses operations of both the protection circuit 148 and the steering circuit 146 and reports the results of such monitoring to the controller 106. The above-described operations of the discharge control circuit 144, the st eering circuit 146, and the protection circuit 148 are controlled by the drive circuit 152 issuing a plurality of drive signals. Operation of the drive circuit 152 is, in turn, controlled by one or more control signals provided by the controller 106.

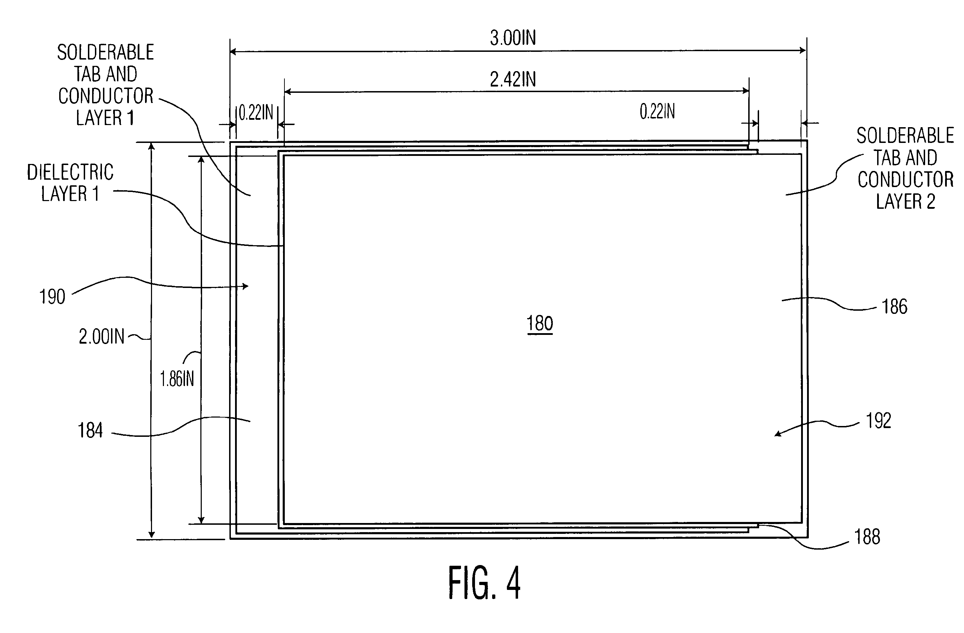

Figure 3 illustrates the energy storage circuit 142 according to an embodiment of the present invention. The energy storage circuit 142 includes a ceramic storage capacitor 160 coupled to an energy conditioning circuit 164. The ceramic storage capacitor 160 is made from a monolithic construction technique and is formed from a ceramic substrate that is used as a construction base. An example of a ceramic capacitor 180 that can be used for the ceramic storage capacitor 160 is illustrated in Figure 4. As shown for the embodiment of Figure 4, the dimensions of the ceramic capacitor 180 are approximately three inches by two inches. The thickness of the ceramic capacitor 180 is approximately one -tenth of an inch. The resulting volume of the ceramic capacitor 180 is less than three -quarters of a cubic inch. The ceramic capacitor 180 is formed from a first conductive layer 184 and a second conductive layer 186, with a dielectric layer 188 disposed between the first and second conductive layers 184, 186. Solderable tabs 190 and 192 are located on opposite sides of the ceramic capacitor 180 to provide a means for electrically connecting the ceramic capacitor 180 to the circuitry of an external defibrillator 100. The solderable tab 190 is formed as an extension of the first conductive layer 184 and the solderable tab 192 is formed as an extension of the second conductive layer 186. Generally, the overlap of the dielectric layer 188 and the conductive layers 184,186 define the capacitive area of the ceramic capacitor 180. Preferably, the dielectric constant of the material from which the dielectric layer 188 is formed has a high K value. Examples of the materials that can be used in forming the dielectric layer 188 include compositions of lead, magnesium lanthanum, zirconium, titanium, and the like. It will be appreciated to one ordinarily skilled in the art, however, that alternative materials currently known, or later developed, can be used as well without departing from the scope of the present invention.

When used in high-voltage applications, such as in external defibrillators, ceramic capacitors exhibit non -linear capacitance characteristics that result in charging and discharging characteristics that are different compared to conventional film or electrolytic capacitors. Additionally, ceramic capacitors lack the stability of conventional film capacitors, especially with respect to temperature. That is, the temperature coefficient of capacitance (TCC) are typically high for ceramic capacitors, causing the capacitance characteristics of ceramic capacitors to vary with temperature. Ceramic capacitors have been employed in implantable defibrillators, as previously mentioned. However, the relatively constant temperature inside the body of a patient, that is, the environment in which the implantable defibrillator operates, mitigates the temperature sensitivity of ceramic capacitors. In contrast to the relatively stable temperature environment of a human body, external defibrillators, especially AEDs, are operated in a variety of temperature conditions. As a result, conventional external defibrillators have not been designed with ceramic capacitors in part due to the difficulties caused by non -linear capacitance characteristics and high TCC.

As previously discussed, the energy storage circuit 142 includes an energy conditioning circuit 164 coupled to the ceramic storage capacitor 160. The energy conditioning circuit 164 conditions the electrical energy discharged from the ceramic storage capacitor 160 accordi ng to a first discharge characteristic by providing the electrical energy from the ceramic storage capacitor 160 in accordance with a second discharge characteristic. As will be explained in more detail below, the second discharge characteristic is controlled by the energy conditioning circuit 164. In this manner, the temperature dependency and non -linear capacitance characteristics of the ceramic storage capacitor 160 can be accommodated, thus allowing ceramic storage capacitors to be used in the externa 1 defibrillator 100.

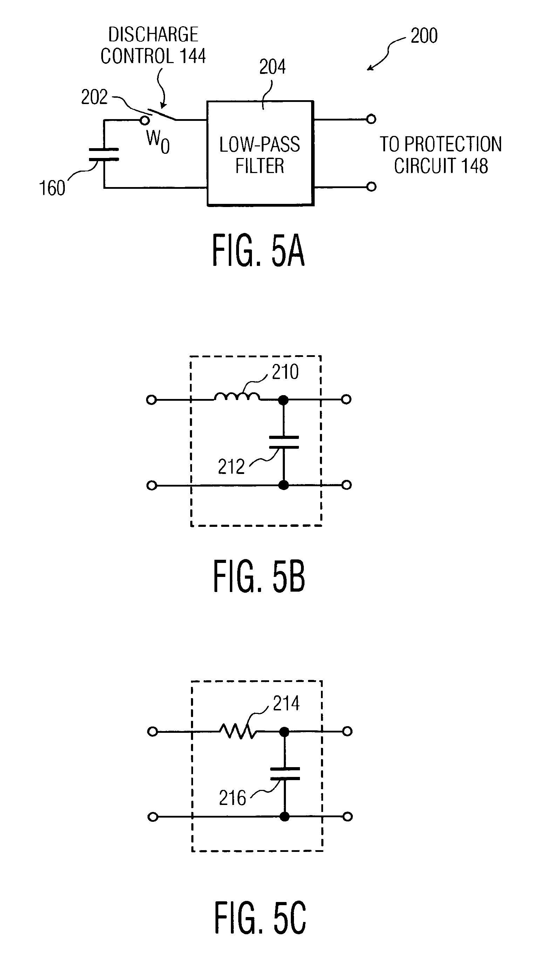

Figure 5A illustrates an energy conditioning circuit 200 that can be used in the energy storage circuit 142. The energy conditioning circuit 200 utilizes a pulse modulation filtering scheme to deliver current -controlled or voltage-controlled discharge waveforms from the ceramic storage capacitor 160. The energy conditioning circuit 200 includes a switch 202 for pulse modulating the electrical energy discharging from the ceramic storage capacitor 160. The switch 202 couples and deco uples the ceramic storage capacitor 160 at a relative high switching frequency ω₀ to form a series of pulses. The switching of the switch 202 can be controlled to provide pulses having various pulse widths and/or various pulse periods. An example of the switching frequency ω₀ of the switch 202 is approximately 30 kHz. However, alternative switching frequencies can also be used. The switching of the switch 202 is controlled by the discharge control circuit 144. As previously discussed, the discharge control circuit 144 controls the discharge of energy stored in the energy storage circuit 142. A low-pass filter 204 is coupled to the switch 202 to filter the waveform resulting from the relatively high-frequency switching of the switch 202. The output energy of the low-pass filter 204 is provided to the protection circuit 148, which in turn transfers the energy to the steering circuit 146, as previously described.

By filtering the energy pulses through the low -pass filter 104, the energy of the ceramic storage capacitor 160 discharging according to a first discharge characteristic can be delivered to the protection circuit according to a second discharge characteristic. The second discharge characteristic can be tailored by programming the discharge control circuit 144 to control the switch 202 to discharge the ceramic storage capacitor 160 through pulses of various widths and pulse periods, as previously mentioned. Figure 5B illustrates a first low-pass filter network including an inductor 210 and a filtering capacitor 212 that can be used in the low -pass filter 204. Figure 5C illustrates a second low-pass filter network including a resistor 214 and a filtering capacitor 216 that can also be used in the low-pass filter 204. Although Figure 5A illustrat es a particular energy conditioning circuit, alternative energy conditioning circuits can be utilized in other arrangements as well.

## Claims

1. An external defibrillator (100) for providing a defibrillating pulse to a patient, comprising:
electrodes (104) for coupling the defibrillating pulse to the patient;
a ceramic storage capacitor (160) for storing electrical energy to be delivered as the defibrillating pulse, the ceramic storage capacitor having an electrical discharge characteristic according to which the stored electrical energy is discharged;
a charging circuit (140) coupled to the ceramic storage capacitor (160) and configured to electrically charge the ceramic storage capacitor;
an energy conditioning circuit (164, 200); a steering circuit (146)
which is coupled to the energy conditioning circuit (162, 200) and the electrodes (104), the steering circuit (146) being configured to couple the electrical energy discharging according to the second discharge characteristic to the electrodes (104) to deliver a defibrillating pulse having a defibrillating pulse characteristic to the patient.
**characterized by that**
the energy conditioning circuit (164, 200) comprises a switch (202) and a low-pass filter coupled to the switch, where the switch is adapted to pulse modulate the electrical energy discharging from the ceramic storage capacitor by coupling and decoupling the ceramic storage capacitor and where the low-pass filter is adapted to filter the waveform resulting from the frequency switching of the switch, the filtering of the energy pulses through the low-pass filter resulting in that the energy of the ceramic storage capacitor discharging according to a first discharge characteristic becomes delivered to a protection circuit (148) according to a second discharge characteristic, where the protection circuit subsequently transfers the energy to the steering circuit (146).

2. The external defibrillator of claim 1 wherein the ceramic storage capacitor (160) comprises a parallel plate ceramic capacitor having a first planar electrode, a second planar electrode, and a planar dielectric disposed between the first and second planar electrodes.

3. The external defibrillator of claim 2 wherein the planar ceramic capacitor has a generally rectangular shape.

4. The external defibrillator of claim 1 wherein the charging circuit (140) comprises a flyback power supply.

5. The external defibrillator of claim 1 wherein the ceramic storage capacitor (160) comprises a ceramic capacitor formed from compositions of at least one of lead, magnesium lanthanum, zirconium, and titanium.

6. The external defibrillator of claim 1 wherein the steering circuit comprises a steering circuit configured to deliver a biphasic defibrillating pulse to the patient.

7. An external defibrillator according to claim 1, wherein the electrodes have an electrode surface to electrically couple to the patient and further having a connector coupled to the electrode surface; where the charging circuit is a high-voltage charging circuit (140); where an energy storage circuit (142) is coupled to the high-voltage charging circuit (140), the energy storage circuit having an output circuit to generate output energy having an output waveform in response to receiving input energy having an input waveform, where the ceramic storage capacitor (160) is coupled to the output circuit and the high-voltage charging circuit; where a switching circuit is coupled to the energy storage circuit and the connector of the electrode, the switching circuit configured to couple the output energy of the output circuit to the patient to provide the defibrillating pulse through the electrode.

8. The external defibrillator of claim 7 wherein the switching circuit comprises a switching circuit configured to deliver a biphasic defibrillating pulse to the patient.

## Patentansprüche

1. Externer Defibrillator (100) zum Abgeben eines Defibrillationsimpulses an einen Patienten, der Folgendes umfasst:
Elektroden (104) zum Weiterleiten des Defibrillationsimpulses zum Patienten,
einen Keramikspeicherkondensator (160) zum Speichern elektrischer Energie, die als Defibrillationsimpuls abzugeben ist, wobei der Keramikspeicherkondensator eine elektrische Entladekennlinie aufweist, gemäß der die gespeicherte elektrische Energie entladen wird,
eine Ladeschaltung (140), die mit dem Keramikspeicherkondensator (160) gekoppelt und so konfiguriert ist, dass sie den Keramikspeicherkondensator elektrisch lädt,
eine Energiekonditionierschaltung (164, 200),
eine Steuerschaltung (146), die mit der Energiekonditionierschaltung (164, 200) und den Elektroden (104) gekoppelt ist, wobei die Steuerschaltung (146) so konfiguriert ist, dass sie die entladene elektrische Energie gemäß der zweiten Entladekennlinie zu den Elektroden (104) weiterleitet, um an den Patienten einen Defibrillationsimpuls mit einer Defibrillationsimpuls-Kennlinie abzugeben,
**dadurch gekennzeichnet, dass**
die Energiekonditionierschaltung (164, 200) einen Schalter (202) und einen mit dem Schalter gekoppelten Tiefpassfilter umfasst, wobei der Schalter so ausgelegt ist, dass er die vom Keramikspeicherkondensator entladene elektrischer Energie pulsmoduliert, indem er den Keramikspeicherkondensator koppelt und entkoppelt, und wobei der Tiefpassfilter so ausgelegt ist, dass er die aus der Frequenzumschaltung des Schalters resultierende Signalform filtert, wobei sich aus der Filterung der Energieimpulse durch den Tiefpassfilter ergibt, dass die Energie, die von dem Keramikspeicherkondensator gemäß einer ersten Entladekennlinie entladen wird, einer Schutzschaltung (148) gemäß einer zweiten Entladekennlinie zugeführt wird, wobei die Schutzschaltung anschließend die Energie zu der Steuerschaltung (146) weiterleitet.

2. Externer Defibrillator nach Anspruch 1, wobei der Keramikspeicherkondensator (160) einen Keramikplattenkondensator mit einer ersten planaren Elektrode, einer zweiten planaren Elektrode und einem planaren Dielektrikum umfasst, das zwischen der ersten und der zweiten planaren Elektrode angeordnet ist.

3. Externer Defibrillator nach Anspruch 2, wobei der planare Keramikkondensator eine im Allgemeinen rechteckige Form hat.

4. Externer Defibrillator nach Anspruch 1, wobei die Ladeschaltung (140) eine Stromversorgung mit Sperrwandler umfasst.

5. Externer Defibrillator nach Anspruch 1, wobei der Keramikspeicherkondensator (160) einen Keramikkondensator umfasst, der aus Zusammensetzungen von mindestens einem der Elemente Blei, Magnesium-Lanthan, Zirkonium und Titan gebildet ist.

6. Externer Defibrillator nach Anspruch 1, wobei die Steuerschaltung eine Steuerschaltung umfasst, die so konfiguriert ist, dass sie einen Zweiphasen-Defibrillationsimpuls an den Patienten abgibt.

7. Externer Defibrillator nach Anspruch 1, wobei die Elektroden eine Elektrodenoberfläche zum elektrischen Weiterleiten zum Patienten und außerdem einen mit der Elektrodenoberfläche gekoppelten Anschluss aufweisen, wobei die Ladeschaltung eine Hochspannungs-Ladeschaltung (140) ist, wobei eine Energiespeicherschaltung (142) mit der Hochspannungs-Ladeschaltung (140) gekoppelt ist, wobei die Energiespeicherschaltung eine Ausgangsschaltung zum Erzeugen einer Ausgangsenergie mit einer Ausgangssignalform als Reaktion auf das Empfangen einer Eingangsenergie mit einer Eingangssignalform aufweist, wobei der Keramikspeicherkondensator (160) mit der Ausgangsschaltung und der Hochspannungs-Ladeschaltung gekoppelt ist, wobei eine Umschaltschaltung mit der Energiespeicherschaltung und dem Anschluss der Elektrode gekoppelt ist, wobei die Umschaltschaltung so konfiguriert ist, dass sie die Ausgangsenergie der Ausgangsschaltung an den Patienten abgibt, um den Defibrillationsimpuls durch die Elektrode zuzuführen.

8. Externer Defibrillator nach Anspruch 7, wobei die Umschaltschaltung eine Umschaltschaltung umfasst, die so konfiguriert ist, dass sie einen Zweiphasen-Defibrillationsimpuls an den Patienten abgibt.

## Revendications

1. Défibrillateur externe (100) pour dispenser une impulsion de défibrillation à un patient, comprenant :
des électrodes (104) pour coupler l'impulsion de défibrillation au patient ;
un condensateur de mémoire céramique (160) pour stocker l'énergie électrique qui doit être délivrée en tant qu'impulsion de défibrillation, le condensateur de mémoire céramique ayant une caractéristique de décharge électrique selon laquelle l'énergie électrique stockée est déchargée ;
un circuit de charge (140) couplé au condensateur de mémoire céramique (160) et configuré pour charger électriquement le condensateur de mémoire céramique ;
un circuit de conditionnement d'énergie (164, 200) ;
un circuit de commande (146) qui est couplé au circuit de conditionnement d'énergie (162, 200) et aux électrodes (104), le circuit de commande (146) étant configuré pour coupler l'énergie électrique se déchargeant selon une deuxième caractéristique de décharge aux électrodes (104) pour délivrer une impulsion de défibrillation ayant une caractéristique d'impulsion de défibrillation au patient ;
**caractérisé en ce que**
le circuit de conditionnement d'énergie (164, 200) comprend un commutateur (202) et un filtre passe-bas couplé au commutateur, où le commutateur est adapté pour moduler en impulsions l'énergie électrique se déchargeant du condensateur de mémoire céramique en couplant et découplant le condensateur de mémoire céramique et où le filtre passe-bas est adapté pour filtrer la forme d'onde résultant de la commutation de fréquence du commutateur, le filtrage des impulsions d'énergie à travers le filtre passe-bas résultant **en ce que** l'énergie du condensateur de mémoire céramique se déchargeant selon une première caractéristique de décharge se trouve déchargée vers un circuit de protection (148) selon une deuxième caractéristique de décharge, où le circuit de protection transfère ensuite l'énergie au circuit de commande (146).

2. Défibrillateur externe selon la revendication 1, dans lequel le condensateur de mémoire céramique (160) comprend un condensateur céramique plan en parallèle ayant une première électrode plan, une seconde électrode plan, et un diélectrique plan disposé entre les première et seconde électrodes plans.

3. Défibrillateur externe selon la revendication 2, dans lequel le condensateur céramique plan a une forme généralement rectangulaire.

4. Défibrillateur externe selon la revendication 1, dans lequel le circuit de charge (140) comprend une alimentation très haute tension par retour-ligne.

5. Défibrillateur externe selon la revendication 1, dans lequel le condensateur de mémoire céramique (160) comprend un condensateur céramique formé à partir de compositions d'au moins le plomb, le magnésium, la lanthane, le zirconium et le titane.

6. Défibrillateur externe selon la revendication 1, dans lequel le circuit de commande comprend un circuit de commande configuré pour délivrer une impulsion de défibrillation biphasique au patient.

7. Défibrillateur externe selon la revendication 1, dans lequel les électrodes ont une surface d'électrode pour coupler électriquement au patient et en outre ayant un connecteur couplé à la surface d'électrode ; où le circuit de charge est un circuit de charge haute tension (140) ; où un circuit de stockage d'énergie (142) est couplé au circuit de charge haute tension (140), le circuit de stockage d'énergie ayant un circuit de sortie pour générer une énergie de sortie ayant une forme d'onde de sortie en réponse à la réception d'une énergie d'entrée ayant une forme d'onde d'entrée, où le condensateur de mémoire céramique (160) est couplé au circuit de sortie et au circuit de charge haute tension ; où un circuit de commutation est couplé au circuit de stockage d'énergie et au connecteur de l'électrode, le circuit de commutation étant configuré pour coupler l'énergie de sortie du circuit de sortie au patient pour fournir une impulsion de défibrillation via l'électrode.

8. Défibrillateur externe selon la revendication 7, dans lequel le circuit de commutation comprend un circuit de commutation configuré pour délivrer une impulsion de défibrillation biphasique au patient.
